# EUROPEAN PATENT APPLICATION

(11) **EP 2 020 231 A1**
(43) Date of publication of application: **04.02.2009**
(21) Application number: 07737035.1
(22) Date of filing: 09.04.2007
(51) Int. Cl.: A61K 31/353, A23L 1/307, A61P 3/06, A61P 9/10

(54) **FAT ABSORPTION INHIBITOR**

(30) Priority: 26.04.2006 JP 2006122645
(71) Applicant: ITO EN, LTD., Tokyo 151-8550 (JP)
(72) Inventor: NOZAWA, Ayumu, Makinohara-shi, Shizuoka 421-0516 (JP); KOBAYASHI, Makoto, Makinohara-shi, Shizuoka 421-0516 (JP); UNNO, Tomonori, Machida-shi, Tokyo 194-0292 (JP)
(74) Representative: Bauch-Koepe, Katharina Anna
(86) International application number: PCT/JP2007/000378
(87) International publication number: WO 2007/125644

(57) **Abstract**

Disclosed is a composition or beverage/food for inhibiting the fat absorption, which is derived from a natural material and therefore is safe, and which can be ingested continuously. The composition or beverage/food comprises the following components: (A) theaflavine monogallate; (B) theaflavine; (C) theaflavine digallate; and (D) polyphenol, wherein these components satisfy the following requirements: (1) a weight-based content ratio [(A)/(B)] is 0.4-100%; (2) the weight of the component (A) is larger than that of the component (C); and (3) a weight-based content ratio [(A)/(D)] is 0.01-1.0%. The composition or beverage/food can be used for the prevention or treatment of a disease such as arteriosclerosis and an ischemic heart disease (e.g., angina pectoris, myocardial infarction).

## Description

### Technical Field

The invention relates to a fat absorption inhibitory composition containing theaflavins as effective ingredients, in particular to a micelle formation inhibitory composition containing theaflavins as effective ingredients, and more particularly to a micellar fat insolubilizing composition, an inhibitory composition from dissolution and uptake of a fat into the micelle, a fat desorption accelerating composition from a micelle, a micelle membrane breaking composition and a composition for accelerating formation of fat precipitation.

### Background Art

Fat has an important role in the body since they are used as materials for forming cell membranes and steroid hormones in addition to protection of the blood vessel, and they are inevitable constituents of the body. However, the chance of excessively taking in the fats is increasing due to satiation of foods in Japan today, and there is accordingly much concern on the risk of diseases caused by the excess intake of fat. The excess intake or metabolic disorder of fat to be caused increases the possibility of evoking various diseases such as arteriosclerosis, ischemic heart disease (angina pectoris, myocardial infarction, etc.). It is also a great social problem because fee for medical treatment increases by the excess intake of fat.

It has been considered as effective for prevention of the excess intake and metabolic disorder of fat to restrict the intake amount of fat and moderately exercise. However, it is difficult to secure enough time for exercise in the busy modern society, and dietary restriction and exercise may sometime accompany pains. Therefore, means for easily controlling the intake of fat is desired. More specifically, a fat absorption inhibitory composition that can be safely and simply ingested is earnestly desired, whereby the composition has an effect to inhibit the fat once ingested in the body from being absorbed in the body, and to excrete the fat out of the body.

A triglyceride itself is directly absorbed into the intestinal epithelium cell, but is decomposed into fatty acid and 2-monoglyceride in the intestinal canal with pancreatic lipase, which form a micelle before being absorbed into the intestinal epithelium cell for the first time. The absorbed fatty acid and 2-monoglyceride are recombined by esterification in the intestinal epithelium cell, and it is taken up into the chylomicron and released into the blood through the lymph duct.

The micelle refers to a spherical structure formed by condensation of plural fats, upon dispersion of the fats in water, with disposing their lipophilic portions to the interior and their hydrophilic portions to the interface between the interior and water. Since a fat has both the hydrophilic portion and lipophilic portion, it naturally forms the micelle. In the body, a bile acid micelle is formed upon contact of bile acid with the fat, which is hydrolysis of triglyceride. The fat is dissolved in the micelle formed, thereby the fat is dissolved into the micelle and such a micellar fat is absorbed into the body through the intestinal epithelium cell.

Pancreatic lipase activity inhibitors from various origins have been developed as the triglyceride absorption suppressing agent, and those known in the art include a lipase inhibitor originating in kale (Japanese Patent No. 3,689,099), a lipase inhibitor originating in rosemary (Japanese Patent No. 3,549,997), a lipase inhibitor containing a yeast fermentation product of persimmon fruit as an effective ingredient (Japanese Patent No. 3,404,235), and a lipase inhibitor containing gallocatechin gallate (GCG) or catechin gallate (CG) as an effective ingredient (Japanese Patent Application Laid-Open (JP-A) No. 2005-247747). However, these inhibitors cannot be expected to exert any effects on the micelles once formed, and almost no reports have been presented for the micelle formation inhibitor compositions as long chain fatty acid decomposition inhibitors. There have not been known on the safe micelle formation inhibitor composition from natural origin, in particular on the fat absorption inhibitory composition having theaflavins that are black tea extract components as effective ingredients, or particularly on the fat absorption inhibitory composition by inhibiting micelle formation.

With respect to the relation between theaflavins and fat, it has been already known, for example, that the blood concentration of cholesterol is controlled by an acceleration action of theaflavin for producing bile acid (Japanese Patent Application Laid-Open (JP-A) No. 2001-302529; the cholesterol level is decreased by a composition containing theaflavins (JP-A No. 2004-155784); and theaflavins have an effect for decreasing blood total cholesterol (TC) level, low density lipoprotein-cholesterol (LDL-C) level and triglyceride (TG) level (Japanese Patent Application National Publication No. 2005-523242). In addition, a lipase inhibitor that includes a lipase inhibitor containing a dimer of flavin-3-ol originating in tea has been already known (WO 2006/004114). However, it has not been known that a composition or beverage that contains theaflavins prescribed to a specified proportion may have an excellent inhibitory action for dissolution of a fat into the micelle, and that these micelle formation inhibitory compositions and beverages may accelerate the excretion without being absorbed into the intestinal epithelium cells. In particular, it has not been known that theaflavin monogallate is excellent in fat absorption inhibitory action among theaflavins, and theaflavin monogallate exhibits a quite excellent effect as compared with green tea extracts when the proportion of theaflavin monogallate is prescribed in a specified amount relative to the amount of total polyphenols.

Patent document 1: Japanese Patent No. 3689099
Patent document 2: Japanese Patent No. 3549997
Patent document 3: Japanese Patent No. 3404235
Patent document 4: JP-A No. 2005-247747
Patent document 5: JP-A No. 2001-302529
Patent document 6: JP-A No. 2004-155784
Patent document 7: Japanese Patent Application National Publication No. 2005-523242
Patent document 8: WO 2006/004114

### Disclosure of Invention

### Problems to be Solved by the Invention

The subject of the present invention is to provide a fat absorption inhibitory composition containing, as effective ingredients, theaflavins that originate in black tea and are safe, a micelle formation inhibitory composition, and food and drink having the effect of above-mentioned compositions.

### Means for Solving the Problem

The inventors of the present invention have found, through intensive studies on various components of natural origin containing fat absorption inhibitory composition, that theaflavins that are components in the black tea have an excellent micelle formation inhibitory action. It was also found that excellent fat absorption inhibitory compositions and food and drink may be obtained by prescribing (1) the proportion of theaflavin monogallate to the total amount of theaflavins (theaflavin monogallate/theaflavins), (2) the proportion of theaflavin monogallate to theaflavin digallate (theaflavin monogallate > theaflavin digallate), and (3) the proportion of theaflavin monogallate to the total amount of polyphenols (theaflavin monogallate/polyphenols), thereby accomplishing the present invention.

Accordingly, the invention relates to:
1. a fat absorption inhibitory composition characterized by containing the following components:
   (A) theaflavin monogallate;
   (B) theaflavins;
   (C) theaflavin digallate; and
   (D) polyphenols,
   wherein these components satisfy the following conditions:
   (1) weight content rate [(A)/(B)] = from 0.4 to 1;
   (2) weight of (A) > weight of (C); and
   (3) weight content ratio [(A)/(D)] = from 0.01 to 1.0;
2. the fat absorption inhibitory composition as set forth in 1 above, further containing one of epigallocatechin gallate and epicatechin gallate, or a combination thereof;
3. the fat absorption inhibitory composition as set forth in 1 or 2 above, characterized by inhibiting micelle formation;
4. a food or drink, characterized by containing the following components:
   (A) theaflavin monogallate;
   (B) theaflavins;
   (C) theaflavin digallate; and
   (D) polyphenols,
   wherein these components satisfy the following conditions:
   (1) weight content rate [(A)/(B)] = from 0.4 to 1;
   (2) weight of (A) > weight of (C); and
   (3) weight content ratio [(A)/(D)] = from 0.01 to 1.0;
5. the food or drink as set forth in 4 above, further containing one of epigallocatechin gallate and epicatechin gallate, or a combination thereof;
6. the food or drink as set forth in 3 or 4 above,
characterized by inhibiting micelle formation.

### Brief Description of the Drawings

FIG. 1 shows the results of researching an action of theaflavins to reduce the intra-micelle cholesterol concentration.
FIG. 2 shows the results of researching an action of digallate-type theaflavin to reduce the cholesterol concentration in the micelles.
FIG. 3 shows the results of researching actions to reduce the cholesterol concentration in the micelles, of theaflavins, green tea extract (trade name: THEAFLAN 90S, manufactured by ITOEN Ltd.) and epigallocatechin gallate.
FIG. 4 shows the results of researching the effects of theaflavins, green tea extract (trade name: THEAFLAN 90S, manufactured by ITOEN Ltd.), epigallocatechin gallate and heat-isomerized catechin, on the concentration of bile acid.
FIG. 5 shows the results of researching actions to reduce the cholesterol concentration in the micelles, of digallate-type theaflavin and catechins (EGCG, ECG, EGCG + ECG).
FIG. 6 shows the results of researching a micelle formation inhibitory effect and component ratio of each of various black tea extract samples.
FIG. 7 shows the composition of theaflavins in each of the green tea extract and black tea extracts used in Embodiment 6.
FIG. 8 shows the compositions of free catechins and gallate-type catechins in each of the green tea extract and black tea extracts used in Embodiment 6.
FIG. 9 shows a relation between the amount of the black tea extract or green tea extract added and the intra-micelle cholesterol releasing ability.
FIG. 10 shows a correlation between the content ratio of theaflavin monogallate to the total amount of polyphenols (MG/Total polyphenol) and residual cholesterol in the micelles.

### Best Mode for Carrying Out the Invention

The fat absorption inhibitory composition of the invention is characterized by containing the following components:
(A) theaflavin monogallate;
(B) theaflavins;
(C) theaflavin digallate; and
(D) polyphenols,
wherein these components satisfy all of the following conditions:
(1) weight content rate [(A)/(B)] = from 0.4 to 1;
(2) weight of (A) > weight of (C); and
(3) weight content ratio [(A)/(D)] = from 0.01 to 1.0. Since epigallocatechin gallate and epicatechin gallate also have a micelle formation inhibitory effect, a more effective fat absorption inhibitory action may be obtained by appropriately adding any one or both of epigallocatechin gallate and epicatechin gallate to the fat absorption inhibitory composition above. While a food or drink that satisfies all the requirements (1) to (3) may be used as it is, one or both of epigallocatechin gallate and epicatechin gallate may also be appropriately added to it. Formulation of the fat absorption inhibitory composition of the present invention is not particularly restricted, and it may be any one of powders, granules, liquids, tablets, liquids, lotions and pastes, for example. Known materials may be appropriately blended into the formulation, which may be prepared by a known method.

The fat absorption inhibitory composition of the present invention may be characterized by satisfying the above-mentioned requirements (1) to (3), and by having a micelle formation inhibitory effect. The micelle formation inhibitory effect as used herein refers to the effect of a composition, etc., having an action to insolubilize the fat to the micelles, an action to inhibit the fat from dissolution and uptake into the micelles, an action to accelerate the release of fat from the micelles, a micelle breaking action, or an action to accelerate precipitation of the fat. It may be of that exhibits single one or a combination of plurality of the above actions.

The fat absorption inhibitory composition of the present invention may be expected more effective when the proportion by weight of polyphenol is in the range from 0.8 to 1.0, more preferably from 0.85 to 1.0, and further preferably from 0.9 to 1.0. The effect of the product according to the present invention may be expectably enhanced when the proportion by weight of free catechin (epigallocatechin, gallocatechin, epicatechin and (+)-catechin) that exhibit no effect according to the present invention is from 0 to 0.12, preferably from 0 to 0.05, and further preferably from 0 to 0.01.

The fat absorption inhibitory composition and the food and drink of the present invention contain (A) theaflavin monogallate formed by allowing one gallate body to link to theaflavin, (B) theaflavin to which no gallate body is linked, and (C) theaflavin digallate formed by allowing two gallate bodies to bonded to theaflavin. The origin of the theaflavins is not particularly restricted, and they may be of natural origin or may be chemically or biologically synthesized. However, in view of safety and availability, the theaflavin is preferably of natural origin, particularly originates in semi-fermentation or fermentation tea, especially in oolong tea or black tea. In a case of originating in semi-fermentation tea or fermentation tea, it may be of any kind of tea tree, and examples of black tea available include Darjeeling tea, Assamese tea, Nilgiri Hills tea, Sikkim tea, Uba tea, Nuwara Eliya tea, Dimbula tea, Uda Pusselewa tea, Kandy tea, Ruhuna tea, Kimen tea, Lapsang Souchong tea, Yunnan tea, Kenya tea, Java tea, Sumatra tea, Nepal tea, Turkish tea and Bangladesh tea. The methods of extraction from natural origins, chemical synthesis and biosynthesis are not particularly restricted, and the methods known in the art may be used.

The fat absorption inhibitory composition and the food and drink of the present invention further contain polyphenols. The polyphenols are not particularly restricted in the invention as long as they are categorized into usual polyphenols such as: flavonoids including catechins, anthocyanin, flavone, isoflavone, flavane and flavanone; phenols including chlorogenic acid; ellagic acid, lignan, curcumin, coumalin and the like. However, in view of the theaflavins originating in black tea, flavonoids are preferable and catechins are particularly preferable. From the known matters that, as described hereinafter, epigallocatechin gallate (EGCG) and epicatechin gallate (ECG) show the fat absorption inhibitory action independent from theaflavins, and that gallocatechin gallate (GCG) and catechin gallate (CG) exhibit a lipase inhibitory action, it is most preferable to appropriately add one or a plurality of these catechins.

Other component than the theaflavins described above may be freely added to the fat absorption inhibitory composition of the present invention. In regard to the component that may be added, one or plural components may be added as long as they do not interfere with the micelle formation inhibitory action of the theaflavins mentioned above. Specific examples of the additives include minerals, materials of plant origin, materials of animal origin, functional materials, vitamins, sweeteners and the like. Since it has been elucidated in the present invention that epigallocatechin gallate and epicatechin gallate also have micelle formation inhibitory actions, although they are restrictive, a more effective micelle formation inhibitory action may be obtained by adding epigallocatechin gallate (EGCG) or epicatechin gallate (ECG).

The fat absorption inhibitory composition of the present invention may be used together with various known lipase inhibitors, and it is thus made possible to obtain more excellent fat absorption inhibitory effect than the known lipase inhibitors. While the lipase inhibitor concomitantly usable is not particularly restricted as long as it does not interfere with the micelle formation inhibitory action of the theaflavins mentioned above, examples of the lipase inhibitor available include those containing gallocatechin gallate (GCG) or catechin gallate (CG) as an effective ingredient.

The fat absorption inhibitory composition of the present invention can be added to and blended with foods or beverages. Viewing that the theaflavins are contained in black tea and are excellent in safety, they are preferably added to and blended with foods or beverages for facilitating continuous uptake of the fat absorption inhibitory composition of the present invention. The object of administration may be any of animals that can enjoy the effect of the fat absorption inhibitory composition of the present invention, including, for example, human, livestock such as cattle, pig, horse and fowl, and pets such as cat, dog and birds. While the method of administration is not particularly restricted in the present invention, oral administration is preferable since it is an easy method.

The food and drink are not particularly restricted in connection with the present invention, and examples thereof include beverages such as non-fruit juice drink, fruit juice drink, vegetable drink, soy milk drink, milk drink, lactic acid drink, tea-base drink, carbonated drink, coffee-base drink, alcoholic drink, mineral-containing drink, vitamin-containing drink and functional food material-containing drinks; dessert foods such as pudding, yogurt, ice cream and jelly; confectioneries such as chocolate, caramel and candy; breads; seasonings such as gravy, source and dressing; snack foods; retort-packed foods; and other instant foods. In terms of absorbency and simplicity, the fat absorption inhibitory composition is preferably added to the beverage.

The food and drink include those for feeding animals. While the food and drink for feeding animals are not particularly restricted, the composition of the present invention may be added to pet foods or beverages for pets.

In blending the fat absorption inhibitory composition of the present invention to the beverage, antioxidants, spices, various esters, organic acids, organic acid salts, inorganic acids, inorganic acid salts, inorganic salts, pigments, emulsifiers, preservatives, seasonings, sweeteners, bitter taste modifiers, acidulants, pH adjustment agents and quality stabilizers may be added alone or in combination. The beverage into which the fat absorption inhibitory agent of the present invention is blended may be provided as a beverage packed in a vessel such as a can, a PET vessel, a paper pack or a bottle.

The amount of the theaflavins added is not particularly restricted in the present invention, and it may be varied depending on the mode of use. If the theaflavins are dissolved in a liquid to use, the concentration is preferably from 1 to 2000 mg/L, more preferably from 10 to 1500 mg/L, and further preferably from 10 to 1000 g/L.

### Embodiments

While the present invention is described in more detail with reference to embodiments, the invention is by no means restricted to the embodiments.

### Embodiment 1: Micellar cholesterol releasability of theaflavins

Theaflavins are classified into four groups of theaflavin (•: no gallate bodies), theaflavin-3-monogallate (■), theaflavin-3'-monogallate (□) and theaflavin-3,3'-digallate (○) depending on the presence or absence of gallate body and the position of the gallate body. Micellar cholesterol releasability was investigated for these four groups of theaflavins.

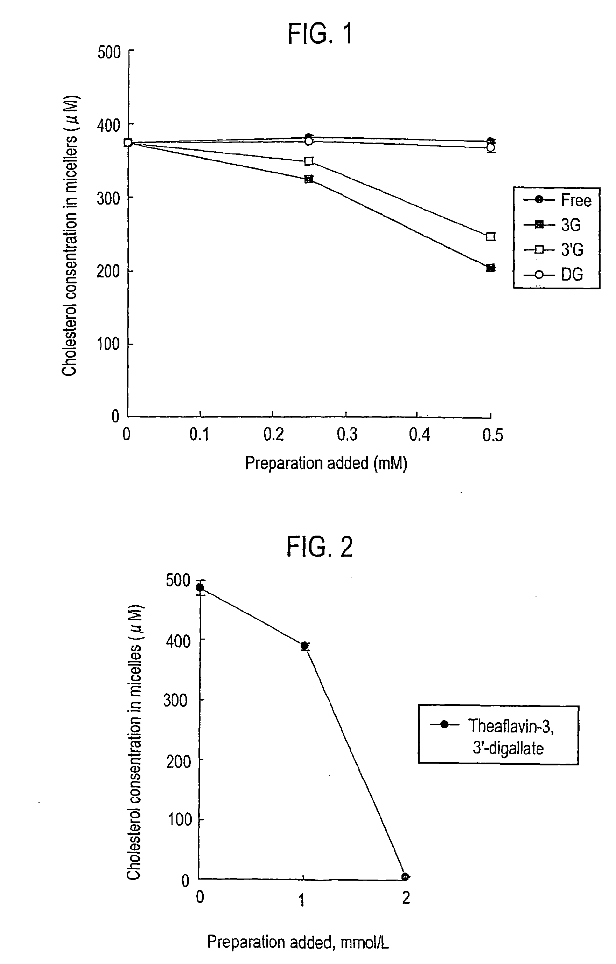

Prepared were micelles composed of 0.5 mM of cholesterol, 6.6 mM of sodium taurocholate, 0.6 mM of phosphatidyl choline of egg yolk origin, 132 mM of sodium chloride (NaCl) and 15 mM of sodium phosphate (NaH₂PO₄·2H₂O). The micelles were incubated for 24 hours at 37°C for stabilizing the micelles. Then 0.1 mL solution of each theaflavin (manufactured by Nagara Science Co.) was added to each of 3 mL portions of the micelles in such a manner that their concentrations were to be respectively 0 µg, 200 µg and 800 µg to 1 mL of the micelles, which were then incubated at 37°C for 1 hour. After that, each of solutions was filtered through a 220 nm filter (manufactured by Whatman Co.) to obtain a clear micelle solution. Cholesterol was extracted from each micelle solution obtained, and the concentration of cholesterol remained in the micelles was measured using a gas chromatograph (manufactured by Shimadzu Corp.) using 5α-cholestane as an internal standard. The results are shown in FIG. 1.

The results showed that, in theaflavin having no gallate and theaflavin-3,3'-digallate (DG), the concentration of cholesterol in the micelles did not change even by raising the amount of addition up to 0.5 mM. On the other hand, the intra-micellar cholesterol concentration, in the case of theaflavin-3-monogallate (3G) and theaflavin-3'-monogallate (3'G), decreased in a manner dependent on the amount of addition, and the concentration of micellar cholesterol at an amount of 0.5 mM addition decreased about twice of the corresponding concentration in the micelles of the case using theaflavin or theaflavin digallate. It was elucidated that monogallate-type theaflavin had an excellent micelle formation inhibitory action among the theaflavins.

### Embodiment 2: Micellar cholesterol release ability of digallate-type theaflavin

Micellar cholesterol release ability of theaflavin-3,3'-digallate (•) was investigated. Since the micellar cholesterol release ability of theaflavin-3,3'-digallate was not distinct in Embodiment 1, the amount of addition was expanded to a range from 0 to 2 mmol/L. The others conditions were similarly applied to the experiment in accordance with the method described in Embodiment 1. The results are shown in FIG. 2.

These results showed that the cholesterol decreasing effect was relatively gentle at the amount of addition of theaflavin-3,3'-digallate up to 1 mmol/L. An excellent micelle formation inhibitory effect was shown by increasing the amount of addition of theaflavin-3,3'-digallate up to 2 mmol/L.

### Embodiment 3: Comparison of theaflavins and catechins

Micellar cholesterol inhibitory activities of theaflavins and catechins were researched. For Theaflavin (•), a theaflavins formulation (manufactured by Funakoshi Corp.) with a proportion of 9.1 of theaflavin, 26.7 of theaflavin monogallate (12.3 of theaflavin-3-monogallate (3G) and 14.4 of theaflavin-3'-monogallate) and 10.0 of theaflavin digallate was prepared to use (Table 1). The purity was 89%.

**Table 1**

| Purity 89% | Proportion |
|---|---|
| Theaflavin | 9.1 |
| Theaflavin monogallate A | 12.3 |
| Theaflavin monogallate B | 14.4 |
| Theaflavin digallate | 10.0 |

### (Method)

Prepared were micelles composed of 0.5 mM of cholesterol (manufactured by Sigma Co.), 6.6 mM of sodium taurocholate (manufactured by Nacalai Tesque Inc.), 0.6 mM of phosphatidyl choline from egg yolk (manufactured by Sigma Co.), 132 mM of sodium chloride (NaCl, manufactured by Nacalai Tesque Inc.) and 15 mM of sodium phosphate (NaH₂PO₄·2H₂O, manufactured by Nacalai Tesque Inc.). The micelles were incubated at 37°C for 24 hours for stabilization. Then 0.1 mL solution of the theaflavins formulation (manufactured by Funakoshi Corp.) was added to each of 3 mL portions of the micelles in such a manner that their concentrations were respectively 0 µg, 200 µg and 800 µg to 1mL micelles, and the solutions were incubated at 37°C for 1 hour (n = 3). Each solution was filtered through a 220 nm filter to obtain a clear solution. Cholesterol was extracted from each micelle solution, and cholesterol remained in the micelle was measured with a gas chromatograph (manufactured by Shimadzu Corp.) using 5α-cholestane as an internal standard.

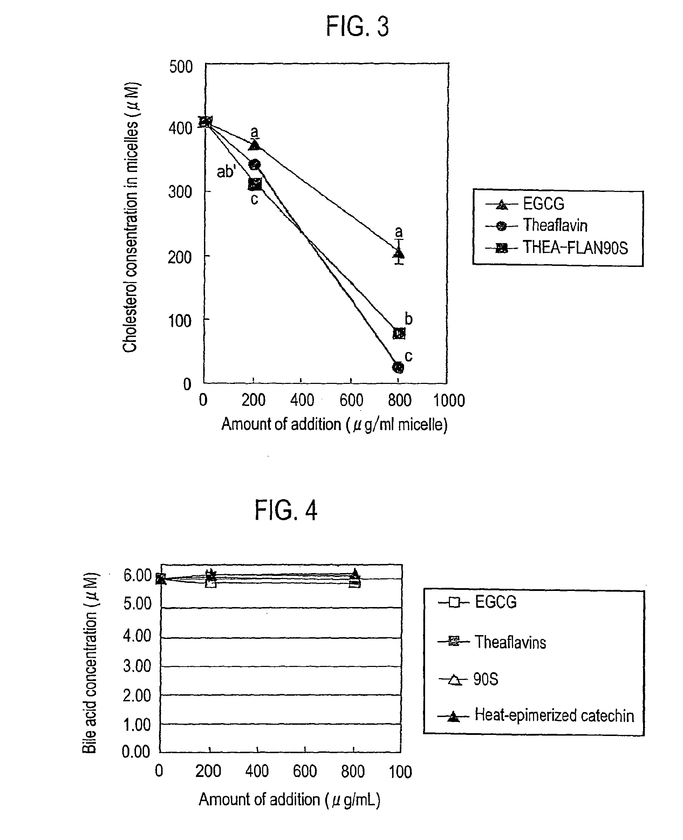

Using a catechin-containing green tea extract (THEAFLAN 90S, manufactured by ITOEN Ltd., ■) or an epigallocatechin gallate formulation (EGCG, manufactured by Wako Pure Chemical Industries, Inc., ▲) instead of the theaflavins used in the above, the experiments were performed in the same method as described above. As a result, the concentration of cholesterol in the micelles decreased by using any one of theaflavins, green tea extract and epigallocatechin gallate, and it was shown that a particularly excellent micelle formation inhibitory effect was exhibited by the addition of the theaflavins in an amount of 500 µg or more per 1 mL of the micelles. The results are shown in FIG. 3.

Moreover, the concentration of bile acid was measured by the enzymatic method in accordance with the process of Eaton et al (Eaton, D. L. et al., Proc. Soc. Exp. Biol. Med. 1976, Jan; 151(1): 198-202). All of the theaflavins, green tea extract and epigallocatechin gallate did not show any effect on the concentration of bile acid. The results are shown in FIG. 4.

### Embodiment 4: Comparison of digallate-type theaflavin and catechins

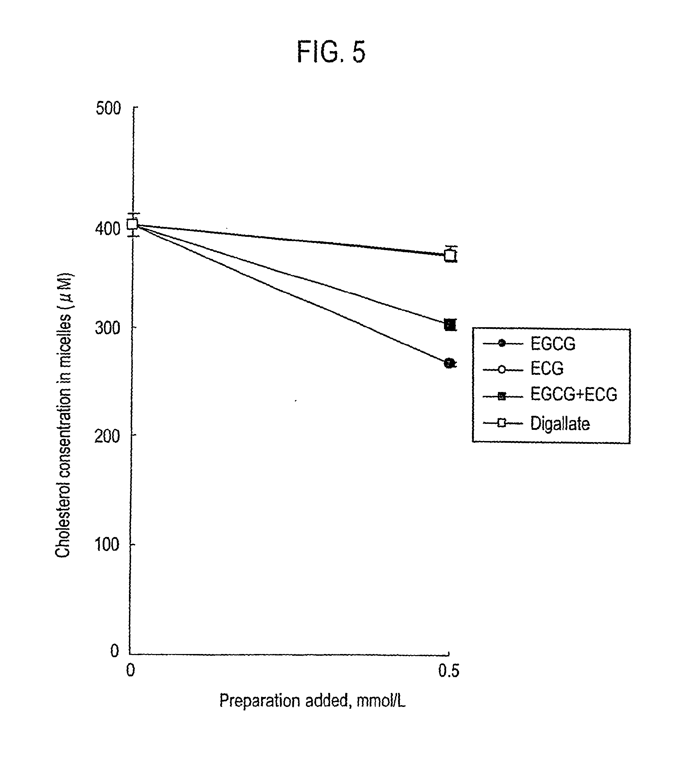

Micellar cholesterol inhibitory abilities of the digallate-type theaflavin and catechins (EGCG, ECG, EGCG + ECG) were researched. The experiments were performed by the same method as in Embodiment 1, except that digallate-type theaflavin (manufactured by Nagara Science Co.) and catechins (EGCG, ECG, and EGCG + ECG, manufactured respectively by Wako Pure Chemical Industries, Inc.) were used. The results are shown in FIG. 5.

The micellar cholesterol concentration was decreased by using either of epigallocatechin gallate (•) or a mixture (■) of epigallocatechin gallate and epicatechin gallate, while digallate-type theaflavin (□) and epicatechin gallate (○) showed little effect on the micellar cholesterol formation.

From the consideration of the results of Embodiments 3 and 4 altogether, it may be understood that digallate-type theaflavin is not responsible for the action to remarkably decrease the micellar cholesterol concentration that has been shown by the theaflavins in Embodiment 3. Further consideration of the result in Embodiment 1 suggests that the action to remarkably decrease the cholesterol concentration in Embodiment 3 may have been given by monogallate-type theaflavin.

### Embodiment 5

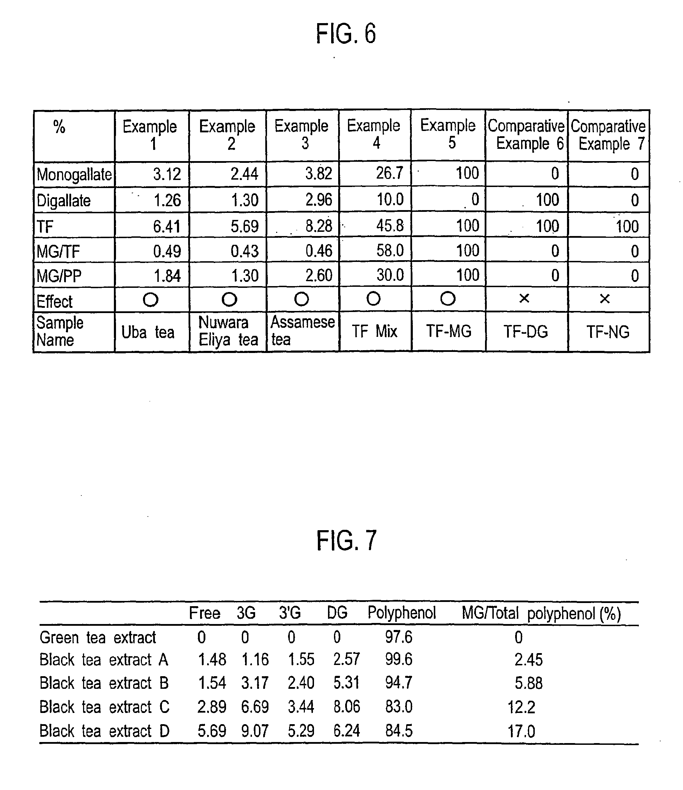

Cholesterol releasing ability of each black tea extract from the bile acid micelles was investigated. The results are shown in FIG. 6. The proportions of theaflavin, theaflavin monogallate, theaflavin digallate and polyphenols were researched to determine a numerical range that was particularly effective for exhibiting the cholesterol releasing ability from the bile acid micelles. The results showed that the most preferable micelle formation inhibitory effect was exhibited when (1) the proportion (MG/TF) of theaflavin monogallate in theaflavins (TF) was in the range from 0.4 to 1, (2) in comparison between the amounts of theaflavin monogallate and theaflavin digallate, the amount of theaflavin monogallate exceeded the amount of theaflavin digallate, and (3) the proportion of theaflavin monogallate in polyphenols, (MG/PP), was in the range from 0.01 to 1.0.

### Embodiment 6:

The effect of change in the proportion of theaflavin monogallate to the total polyphenols in the black tea extract that was given to the micellar cholesterol release ability was investigated.

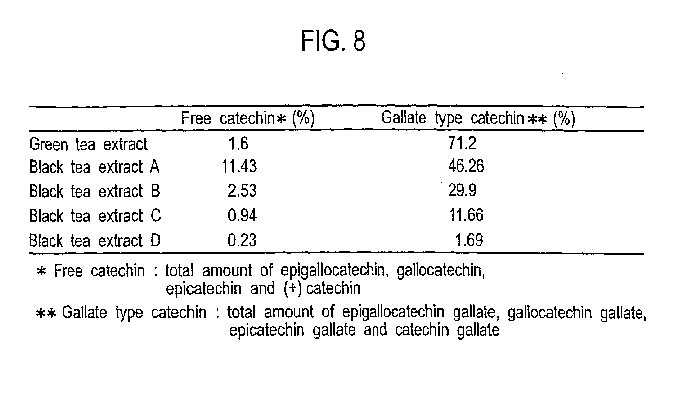

A test extract of black tea was prepared by extraction of 10 g of black tea leaves immersed in 30 mL of 60% ethanol. The immersion extraction process had repetition of two extraction steps each for 30 minutes at room temperature. Such process was applied to 4 kinds of black tea leaves to obtain 4 kinds of test extracts of the black tea (samples A to D). Each extract obtained was filtered by suction filtration (filter paper No. 2, 90 mm, manufactured by ADVANTECH Co.), adsorption-partition chromatography (column: HW-40EC, manufactured by TOSOH Corp.) was performed by eluting with 60% ethanol to retrieve the fractions of 3rd to 5th bets, and the collected fractions were freeze-dried. The compositions of the black tea extracts A to D and of green tea extract (Trade name: THEAFLAN 90S, manufactured by ITOEN Ltd.) are shown in FIGs. 7 and 8. In FIG. 7, "free" denotes free theaflavin, "3G" denotes theaflavin-3-monogallate, "3'G" denotes theaflavin-3'-monogallate, "DG" denotes theaflavin-3,3'-digallate, "Polyphenol" denotes total polyphenols, and "MG/Total polyphenol (%)" denotes the content ratio of theaflavin monogallate to the total polyphenols. Here, theaflavin monogallate refers to a compound having one gallate group linked to the basic structure of theaflavin, and specifically includes theaflavin-3-monogallate (3G) and theaflavin-3'-monogallate (3'G). In FIG. 8, free catechin denotes a total amount of epigallocatechin, gallocatechin, epicatechin and (+)catechin, and gallate-type catechin denotes the total amount of epigallocatechin gallate, gallocatechin gallate, epicatechin gallate and catechin gallate.

Next, micelles for test were prepared (pH 6.8) at a composition of 0.5 mM of cholesterol, 6.6 mM of sodium taurocholate, 0.6 mM of egg yolk phosphatidyl choline, 132 mM of sodium chloride (NaCl) and sodium phosphate (NaH₂PO₄·2H₂O), and the micelles were incubated at 37°C for 24 hours for stabilization.

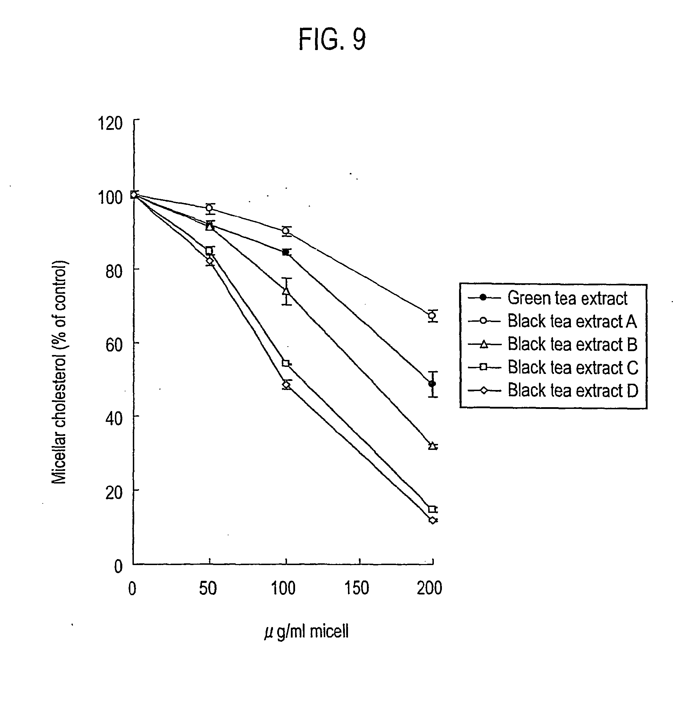

To each of 3 mL portions of the micelles obtained, 0.1 mL of the black tea extract (samples A to D) or green tea extract (THEAFLAN 90S solution, manufactured by ITOEN Ltd.) was added respectively so that their concentrations were respectively 50 µg/mL, 100 µg/mL and 200 µg/mL to the micelles, and each of the mixed solutions was then incubated at 37°C for 1 hour. The solution was filtered through a filter (220 nm) after the incubation, and extraction of the fat, saponification and extraction with hexane (3 times) were performed before trimethylsilylation (BSTFA + TMCS, manufactured by SUPELCO Co.). The amount of cholesterol remained in the micelles was measured by GC using 5α-cholestane as an internal standard. The results are shown in FIG. 9.

FIG. 9 shows that the micellar cholesterol release ability increases according as the amount of addition of the black tea extract or green tea extract increases. According to this matter, it is understood that the black tea extract and green tea extract have the fat absorption inhibitory action that results from inhibition of the micellar formation. FIG. 9 also shows that the micellar cholesterol release ability increases according as the content ratio of theaflavin monogallate to the total polyphenols amount (MG/total polyphenol) increases.

FIG. 9 also shows that theaflavins do not naturally improve the cholesterol release ability, but exhibits excellent cholesterol release ability only when they are added in a specified amount or more.

Specifically, the cholesterol release ability of sample A (MG/Total polyphenol (%) = 2.45) was quite weaker than the cholesterol release ability of the green tea extract (MG/Total polyphenol (%) = 0). However, the cholesterol release abilities of sample B (MG/total polyphenol (%) = 5.88), sample C (MG/Total polyphenol (%) = 12.2) and sample D (MG/total polyphenol (%) = 17.0) were quite stronger than the cholesterol release ability of the green tea extract (MG/Total polyphenol (%) = 0). Moreover, the cholesterol release ability of theaflavins became stronger depending on the content ratio of theaflavin monogallate to total polyphenols amount (MG/Total polyphenol).

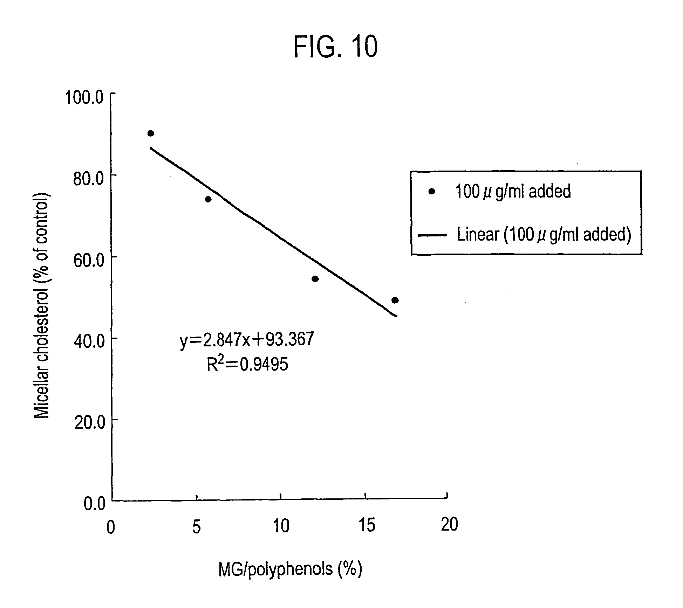

Moreover, the content ratio of theaflavin monogallate to total polyphenols amount (MG/Total polyphenol), at which the concentration of micellar cholesterol remained when 100 µg/ml (micelle) of the theaflavin were added was the same as that of the green tea extract, was calculated. As a result, it was confirmed that more excellent effect than that of the green tea extract was exhibited at a ratio of MG/Total polyphenol of about 4 or more. It was also confirmed that excellent cholesterol release ability twice or more of that of the green tea extract was exhibited in the range from 10 to 20% of the content ratio of theaflavin monogallate to the total polyphenols amount (MG/total polyphenol). The results are shown in Fig. 10.

### Industrial Applicability

According to the present invention, the fat absorption inhibitory composition and the food and drink that are naturally occurring and safe are provided. Such fat absorption inhibitory composition and food and drink are useful for prevention and therapy of diseases such as arteriosclerosis and ischemic heart disease (such as angina pectoris and myocardial infarction). Since the article of the present invention is usable together with various lipase inhibitors, more efficient prevention and therapy of the above-mentioned diseases may be expected than that using conventional lipase inhibitor alone.

## Claims

1. A fat absorption inhibitory composition comprising the following components:
(A) theaflavin monogallate;
(B) theaflavins;
(C) theaflavin digallate; and
(D) polyphenols,
wherein said components satisfying the following conditions:
(1) weight content rate [(A)/(B)] = from 0.4 to 1;
(2) weight of (A) > weight of (C); and
(3) weight content ratio [(A)/(D)] = from 0.01 to 1.0.

2. The fat absorption inhibitory composition as set forth in Claim 1, further comprising either of epigallocatechin or epicatechin gallate, or the combination thereof.

3. The fat absorption inhibitory composition as set forth in Claim 1 or 2, **characterized by** inhibiting micelles formation.

4. A food or drink, comprising the following components:
(A) theaflavin monogallate;
(B) theaflavins;
(C) theaflavin digallate; and
(D) polyphenols,
wherein said components satisfying the following conditions:
(1) weight content rate [(A)/(B)] = from 0.4 to 1;
(2) weight of (A) > weight of (C); and
(3) weight content ratio [(A)/(D)] = from 0.01 to 1.0.

5. The food or drink as set forth in Claim 4, further comprising either of epigallocatechin or epicatechin gallate, or the combination thereof.

6. The food or drink as set forth in Claim 3 or 4, **characterized by** inhibiting micelle formation.
